# EUROPEAN PATENT SPECIFICATION

(11) **EP 1 795 160 B1**
(45) Date of publication and mention of the grant of the patent: **06.05.2009**
(21) Application number: 05026730.1
(22) Date of filing: 07.12.2005
(51) Int. Cl.: A61F 11/08

(54) **Use of a hearing protection device with acoustic filter element**
Verwendung einer Gehörschutzvorrichtung mit einem akustischen Filter
Utilisation d'un dispositif de protection auditive avec un filtre acoustique

(43) Date of publication of application: 13.06.2007
(60) Divisional application: 09151812.6
(73) Proprietor: Phonak AG, 8712 Stäfa (CH)
(72) Inventor: Oberdanner, Hannes, 8633 Wolfhausen (CH)
(74) Representative: Schwan - Schwan - Schorer

(56) References cited:
- EP-A- 1 046 382
- WO-A-97/18779
- FR-A- 2 095 127
- US-A1- 2003 133 583

## Description

The present invention relates to a use of a hearing protection device comprising a shell to be worn at least partially within a user's ear canal and an acoustic filter element for attenuating sound according to the preamble of claim 1.

Generally, hearing protection devices may have a generic shape and be made of a soft material for adapting to the individual shape of the user's outer ear and ear canal or they may have a customized shape which is individually adapted to the shape of the user's outer ear and ear canal in the manufacturing process by measuring the shape of the user's outer ear and ear canal prior to manufacturing. In the latter case, the hearing protection devices may be made of a relatively hard material.

In order to be able to control the acoustic attenuation attained by the hearing protection device at least to some extent, hearing protection devices frequently include a passive acoustic filter. Such acoustic filter serves to reduce the maximum attainable acoustic attenuation in a defined manner by providing an acoustic by-pass to the shell or body of the hearing protection device.

One type of acoustic filters is known as tube filters or vent filters and essentially consists of a narrow tube which is designed such that the filter has a cut-off frequency below which the acoustic attenuation usually has a steep linear decrease. While with tube filters good variation of the average attenuation is obtainable, they inherently have much lower attenuation at low frequencies compared to higher frequencies. Further, in total, attenuation of tube filters is significantly non-linear over the audible frequency range. An example of tube filters can be found in US 5,832,094.

Another type of acoustic filters is known as membrane filters which comprise at least one sheet-like membrane placed within a sound channel. Membrane filters usually have a more linear attenuation than tube filters, but the achievable variability of the average attenuation (i.e. the attenuation averaged over the audible frequency range) is usually much lower than for tube filters. In particular, the practically available materials are not able to achieve high average attenuation. Further, membrane filters are difficult to manufacture. An example of membrane filters can be found in WO 01/76520 A1.

US 5,799,658 relates to a hearing protection earplug comprising a body made of a foam material into which a shank made of a porous material is inserted which projects beyond the outer end of the foam body in order to provide for a handle for handling the earplug manually. However, the shank made of porous material does not penetrate through the inner end of the body made of foam material. It is also mentioned in US 5,799,658 that the porous shank may serve as an embedded acoustic filter.

DE 41 19 615 A1 relates to an earplug comprising a shell forming a hollow chamber which is filled with a particulate material serving for sound attenuation. At the outer end and at the inner end the shell is provided with a sound opening.

DE 1 096 545 relates to an earplug comprising a closed shell which is filled with balls.

US 2,785,675 relates to an earplug comprising a hollow shell having sound openings at its inner end and at its outer end, which is filled with a cellular fibrous material such as milk weed floss.

WO 97/18779 A1 relates to a hearing protection device according to the preamble of claim 1, wherein the acoustic filter element acts as a pressure regulator with a slow leak rate. The , pressure regulator is located within a silicone tubing which is connected via an adapter and a plastic cone to the shell of the earplug.

FR 2.095.127 relates to a hearing protection earplug comprising a shell having a cavity filled with a filling material which is permeable to air. The cavity is closed by an insert comprising an opening. The sound attenuation provided by the earplug may be adjusted according to the user's needs by reducing or increasing the amount of filling material in the cavity.

It is an object of the invention to provide for a use of a hearing protection device having an acoustic filter element, wherein both relatively linear attenuation and relatively high variability of the average attenuation can be achieved and wherein the hearing protection device is provided with such a filter element, which allows adaptation of the hearing protection device to different sound/noise situations.

This object is achieved by a use of a hearing protection device as defined in claim 1

In general, the invention is beneficial in that, by providing the acoustic filter element with an open-pore porous material, a more linear attenuation than with tube filters can be achieved, while higher variability of the average attenuation than with membrane filters can be achieved. In addition, better control of the average attenuation and hence less variance of the attenuation can be achieved than with membrane filters. In addition, manufacturing is easier and more reproducible than with membrane filters.

The invention is particularly beneficial in that, by engaging the filter element with the shell in a detachable manner, the filter element can be easily exchanged. Thereby the user may adapt the attenuation provided by the hearing protection device to the actual sound/noise environment, so that, for example, at low noise levels overprotection can be avoided and hence communication can be improved, whereby, for example, the motivation to actually wear the hearing protection device could be improved. Another benefit is the option to test and select the filter with the most appropriate attenuation characteristic according to the individual preference of the user. For example, the user could test different filters and finally could select the filter which appears to be the most appropriate one.

In an open-pore porous filter sound energy is dissipated by friction of the air molecules moving with a certain velocity within the pores. The pores have to be open, because otherwise the sound energy could not penetrate into the porous material. The porous material may have a pore size of from 1 to 500 µm, a porosity of from 0.05 to 0.90 (the porosity is defined as the ratio of the open air volume to the total volume), a structural index of from 1 to 12 (the structural index is defined as the ratio of the open air volume to the effective porous volume), and a flow resistance per unit length of from 0.1 to 1000 Pa s/m² (the flow resistance per unit length is defined as the ratio of the specific flow resistance to the layer thickness in the flow direction, with the specific flow resistance being defined as the ratio of the pressure difference across the material to the velocity of the air flow).

The porous material may comprise at least one of a foamed material, a sintered material, a fibre material, a particulate material, a fleece material, a braided material and a woven material.

The porous material may made of at least one of a plastic material, ceramic material, a metal, an alloy, and a glass material.

The acoustic filter element may comprise a first portion and a second portion which differ from each other regarding structure, in particular, regarding pore size and/or grain size.

The acoustic filter element may have a tube-like shape, and it may comprise at least one channel extending from the outer end to the inner end of the acoustic filter element.

The shell may be produced by an additive layer-by-layer build-up process, such as selective laser sintering of a powder material, for example, a polyamide powder material, whereby the outer shape of the shell is designed according to the previously measured inner shape of the user's outer ear and ear canal. An overview over additive layer-by-layer build-up processes is given, for example, in US 6,533,062 B1 or US 2003/0233583 A1. Such processes are also called "rapid prototyping".

The housing of the acoustic filter element may have an end open towards ambience and an end open towards an inner opening of the receptacle. The housing may be provided at both open ends with an air-permeable membrane for protection of the porous material.

The acoustic filter element may be engaged with the shell by a snap-in connection, a bayonet connection, an adhesive connection, or an interference fit.

In the following, examples of the invention will be illustrated by reference to the attached drawings.
Fig. 1 is a schematic cross-sectional view of a first embodiment of a hearing protection device according to the invention as worn by a user;
Fig. 2 is a schematic cross-sectional view of a hearing protection device which is not covered by the invention;
Fig. 3 is a view like Fig. 2, with an embodiment being shown which is not covered by the invention; and
Fig. 4 is a view like Fig. 2, with another embodiment being shown which is not covered by the invention.

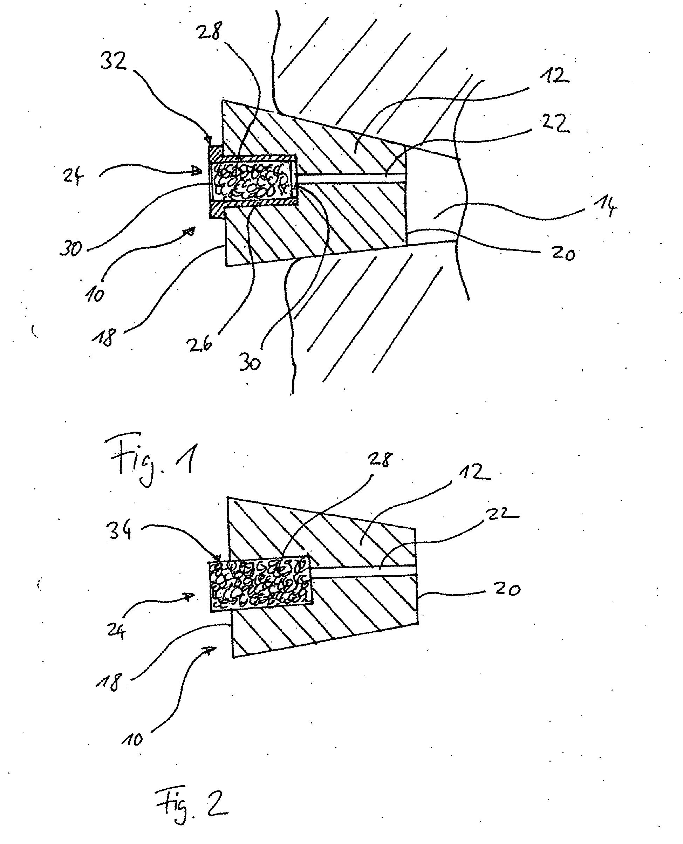

Fig. 1 is a schematic cross-sectional view of a hearing protection earplug 10 comprising a shell 12 which is to be inserted into the user's ear canal 14 at least partially. Preferably the shell 12 is customized, i.e. it has an outer shape individually defined according to the measured inner shape of the user's outer ear and ear canal 14 (which can be measured, for example, by direct laser scanning of the ear or by laser scanning of an impression of the ear). The shell 12 may be produced according to the obtained individual ear shape data by an additive layer-by-layer build-up process, such as laser sintering of a powder material, such as a polyamide powder material. Alternatively, the layer-by-layer build-up process may be a three-dimensional printing, i.e. "ink-jet"-like process.

The shell 12 is formed with a receptacle 16 which is open towards the outer end 18 of the shell 12 and towards the inner end 20 of the shell 12, and hence the ear canal 14, via a sound passage 22.

The earplug 10 further comprises an acoustic filter element 24 for attenuating sound which is engaged with the receptacle 16 in a detachable manner. In the embodiment shown in Fig. 1, the filter element 24 completely fills the receptacle 16, with the inner end of the filter element 24 extending to the sound passage 22 and the outer end of the filter element 24 projecting outwardly beyond the outer end 18 of the shell 12 towards the ambience.

The filter element 24 comprises a housing 26 containing open-pore porous material 28. The housing 26 could be made, for example, of a plastics material or a metal or alloy. The housing 26 may have a tube-like shape and may have an inner and an outer open end which both are provided with an air-permeable membrane 30 for protecting the porous material 28. The porous material 28 may comprise a foamed material, sintered material, a fibre material, a particulate material, a fleece material, a braided material or a woven material and may be made of plastics, ceramics, metals, alloys and/or glass. Examples of such materials are foamed plastics, sintered polymers, foamed ceramics, sintered glass, sintered metal, mineral wool, rock wool, glass wool, kaolin wool, cotton, polycarbonate fibre, basalt wool, aluminium wool or wood fibre; woven material, braided material or fleece material made of metal, ceramic, synthetic fibres or natural fibres; sintered brass, cork, thermoplastic elastomeric materials, and/or polyurethane.

The pore size preferably is from 1 to 500 µm, the porosity is preferably from 0.05 to 0.90, the structural index preferably is from 1 to 12 and the flow resistance per unit length preferably is from 0.1 to 1000 Pa s/m².

The porous material 28 may be homogenous or it may be inhomogeneous in that it may comprise a plurality of different portions which differ from each other regarding structure, such as pore size and/or grain size, or regarding the material as such.

The parameters and the material/composition of the porous material 28, as well as the spatial arrangement thereof, can be used to tailor the desired acoustic attenuation provided by the filter element 24.

The filter element 24 is provided with an outwardly projecting portion 32 which can be manually operated in order to remove the filter element 24 from the shell 12 for replacement by an acoustic filter element of the same type or of a different type with different attenuation characteristic. The releasable engagement of the filter element 24 with the receptacle 16 can be realized, for example, by a bayonet-like connection, a snap-in connection, or an interference fit. Alternatively, the releasable engagement could be achieved by a relatively weak adhesive connection. In this case, the adhesive would have to be replaced when replacing the acoustic filter element 24.

Fig. 2 shows an embodiment which is not covered by the invention, wherein the housing 26 of the acoustic filter element 24 has been omitted so that the open-pore porous material 28 is directly engaged within the receptacle 16, for example, by elastic deformation of the porous material 28 or by an adhesive material. The porous material 28 is provided with an outwardly projecting portion 34 which enables the porous material 28 being manually removed from the receptacle 16 for replacement.

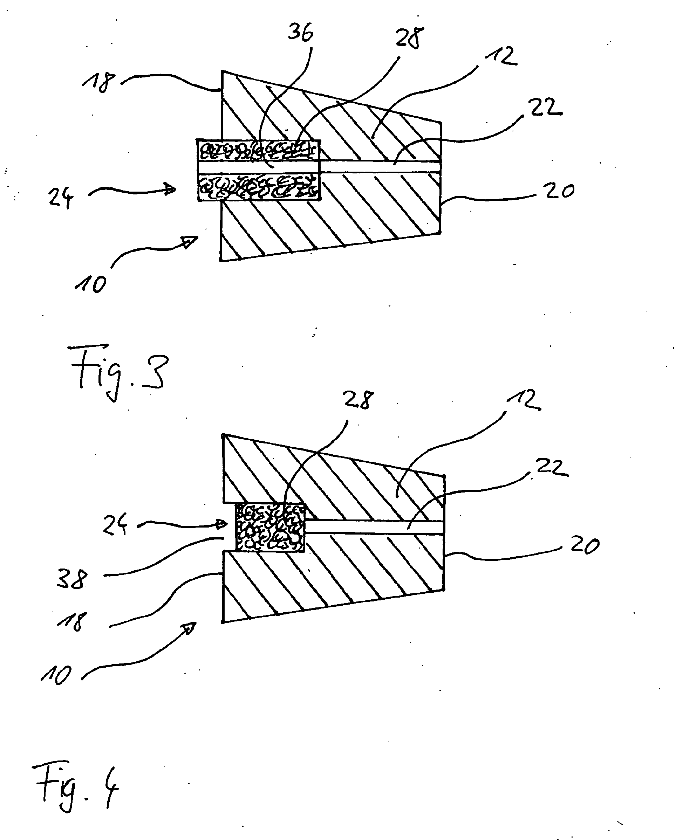

Fig. 3 shows an embodiment which is not covered by the invention, wherein the acoustic filter element 24, and hence the porous material 28, is provided with at least one channel 36 extending from the outer end to the inner end of the filter element 24. In this case, the filter element 24 may look like a hollow tube.

Fig. 4 shows an embodiment which is not covered by the invention wherein the acoustic filter element 24 has been formed integral with the shell 12 during manufacturing of the shell 12. In this case the filter element 24 likewise has an open-pore porous structure and extends between the sound passage 22 and an outer opening 38 of the shell 12. If the shell 12 is produced by laser sintering of a powder material, the porous structure of the acoustic filter element 24 can be produced by reducing the total laser energy provided per unit volume compared to the production of the remainder of the shell 12. Thereby the powder particles would not melt completely, thereby leaving an interconnected void volume between the sintered particles of the powder material.

In an alternative approach, the open-pore porous structure of the filter element 24 could be realized with a pre-defined geometry. In this case, three-dimensional printing, i.e. "ink-jet"-like processes would be the most appropriate ones.

## Claims

1. A use of a hearing protection device (10) comprising a shell (12) to be worn at least partially within a user's ear canal (14), said shell being provided with a receptacle (16) having an outer opening towards the ambience and an inner opening (22) towards the ear canal, and an acoustic filter element (24) for attenuating sound comprising a housing (26) containing open-pore porous material (28), **characterized by** the filter element being engaged within the the receptacle in a detachable manner and by the use comprising: removing the acoustic filter element from the shell and engaging another acoustic filter element comprising open-pore porous material with the shell, wherein the latter acoustic filter element has a sound attenuation characteristic different from that of the previous acoustic filter element.

2. The use of claim 1, wherein the housing (26) has an end open towards ambience and an end open towards the inner opening (22) of the receptacle (16).

3. The use of claim 2, wherein the housing (26) is provided at both open ends with an air-permeable membrane (30) for protection of the porous material (28).

4. The use of one of the preceding claims, wherein the porous material (28) comprises at least one of a foamed material, a sintered material, a fiber material, a particulate material, a fleece material, a braided material and a woven material.

5. The use of claim 4, wherein the porous material (28) is made of at least one of a plastic material, ceramic material, a metal, an alloy, and a glass material.

6. The use of one of the preceding claims, wherein the acoustic filter element (24) is engaged with the shell (12) by a snap-in connection, a bayonet connection, an adhesive connection, or an interference fit.

7. The use of one of the preceding claims, wherein the porous material (28) comprises a first portion and a second portion which differ from each other regarding material and/or structure.

8. The use of claim 7, wherein the first and the second portion differ regarding pore size and/or grain size.

9. The use of one of the preceding claims, wherein the acoustic filter element (24) has a tube-like shape.

10. The use of claim 9, wherein the acoustic filter element (24) is provided with at least one channel (36) extending from the outer end to the inner end of the acoustic filter element.

11. The use of one of the preceding claims, wherein the porous material (28) has a pore size from 1 to 500 µm.

12. The use of one of the preceding claims, wherein the porous material (28) has a porosity from 0.05 to 0.90.

13. The use of one of the preceding claims, wherein the porous material (28) has a structural index from 1 to 12.

14. The use of one of the preceding claims, wherein the porous material (28) has a flow resistance per unit length of 0.1 to 1000 Pa·s/m².

## Patentansprüche

1. Verwendung einer Gehörschutzvorrichtung (10) mit einer Schale (12), welche mindestens zum Teil im Gehörgang (14) eines Nutzers zu tragen ist, wobei die Schale mit einer Aufnahme (16) mit einer der Umgebung zugewandten äußeren Öffnung und einer dem Gehörgang zugewandten inneren Öffnung (22) versehen ist, sowie einem akustischen Filterelement (24) zur Schalldämpfung mit einem Gehäuse (26), welches offenporiges Material (28) enthält, **dadurch gekennzeichnet, dass** das Filterelement mit der Aufnahme in lösbarem Eingriff steht, sowie **dadurch**, dass im Zuge der Verwendung das akustische Filterelement von der Schale entfernt wird und ein anderes akustisches Filterelement mit offenporigem Material mit der Schale in Eingriff gebracht wird, wobei dieses akustische Filterelement eine Schalldämpfungscharakteristik aufweist, die sich von derjenigen des vorherigen akustischen Filterelements unterscheidet.

2. Verwendung gemäß Anspruch 1, wobei das Gehäuse (26) ein zur Umgebung hin offenes Ende und ein zu der inneren Öffnung (22) der Aufnahme (16) hin offenes Ende aufweiset.

3. Verwendung gemäß Anspruch 2, wobei das Gehäuse (26) an beiden Enden mit einer luftdurchlässigen Membran (30) für den Schutz des porösen Materials (28) versehen ist.

4. Verwendung gemäß einem der vorhergehenden Ansprüche, wobei das poröse Material ein geschäumtes Material, ein gesintertes Material, ein Fasermaterial, ein aus Teilchen bestehendes Material, ein Vlies-Material, ein geflochtenes Material und/oder ein gewebtes Material aufweist.

5. Verwendung gemäß Anspruch 4, wobei das poröse Material (28) aus Kunststoffinaterial, Keramikmaterial, einem Metall, einer Legierung und/oder einem Glasmaterial hergestellt ist.

6. Verwendung gemäß einem der vorhergehenden Ansprüche, wobei das akustische Filterelement (24) mit der Schale (12) mittels einer Schnappverbindung, einer Bajonettverbindung, einer Klebeverbindung oder einer Presspassung in Eingriff steht.

7. Verwendung gemäß einem der vorhergehenden Ansprüche, wobei das poröse Material (28) einen ersten Teil und einen zweiten Teil aufweist, die sich hinsichtlich Material und/oder Struktur unterscheiden.

8. Verwendung gemäß Anspruch 7, wobei sich der erste und der zweite Teil hinsichtlich der Porengröße und/oder der Korngröße unterscheiden.

9. Verfahren gemäß einem der vorhergehenden Ansprüche, wobei das akustische Filterelement (24) eine röhrenartige Form aufweist.

10. Verwendung gemäß Anspruch 9, wobei das akustische Filterelement (24) mindestens einen Kanal (36) aufweist, der sich von dem äußeren Ende zu dem inneren Ende des akustischen Filterelements erstreckt.

11. Verwendung gemäß einem der vorhergehenden Ansprüche, wobei das poröse Material (28) eine Porengröße zwischen 1 und 500 µm aufweist.

12. Verfahren gemäß einem der vorhergehenden Ansprüche, wobei das poröse Material (28) ein Porosität von 0.05 bis 0,90 aufweist.

13. Verwendung gemäß einem der vorhergehenden Ansprüche, wobei das poröse Material (28) einen Strukturindex von 1 bis 12 aufweist.

14. Verwendung gemäß einem der vorhergehenden Ansprüche, wobei das poröse Material (28) einen Strömungswiderstand pro Einheitslänge von 0,1 bis 1000 Pa·s/m² aufweist.

## Revendications

1. Utilisation d'un système de protection auditive (10) comportant une coque (12) devant être portée au moins partiellement à l'intérieur d'un canal auditif (14) d'un utilisateur, ladite coque étant pourvue d'un réceptacle (16) ayant une ouverture extérieure vers le milieu ambiant et une ouverture intérieure (22) vers le canal auditif, et un élément à filtre acoustique (24) destiné à atténuer les sons comportant un boîtier (26) contenant une matière poreuse (28) à pores ouverts, **caractérisée en ce que** l'élément à filtre est engagé à l'intérieur du réceptacle d'une manière détachable, et **en ce que** l'utilisation comprend : l'enlèvement de l'élément à filtre acoustique de la coque, l'engagement d'un autre élément à filtre acoustique, comprenant une matière poreuse à pores ouverts, avec la coque, le dernier élément à filtre acoustique ayant une caractéristique d'atténuation des sons différentes de celle de l'élément de filtre acoustique précédent.

2. Utilisation selon la revendication 1, dans laquelle le boîtier (26) a une extrémité ouverte vers le milieu ambiant et une extrémité ouverte vers l'ouverture intérieure (22) du réceptacle (16).

3. Utilisation selon la revendication 2, dans laquelle le boîtier (26) est pourvu, aux deux extrémités ouvertes, d'une membrane (30) perméable à l'air pour la protection de la matière poreuse (28).

4. Utilisation selon l'une des revendications précédentes, dans laquelle la matière poreuse (28) comprend au moins l'une d'une matière à l'état de mousse, d'une matière frittée, d'une matière fibreuse, d'une matière en particules, d'une matière laineuse, d'une matière tressée et d'une matière tissée.

5. Utilisation selon la revendication 4, dans laquelle la matière poreuse (28) est formée d'au moins l'une d'une matière plastique, d'une matière céramique, d'un métal, d'un alliage et d'une matière du type verre.

6. Utilisation selon l'une des revendications précédentes, dans laquelle l'élément à filtre acoustique (24) est engagé avec la coque (12) par une liaison par encliquetage, une liaison par baïonnette, une liaison par adhésif ou un ajustement serré.

7. Utilisation selon l'une des revendications précédentes, dans laquelle la matière poreuse (28) comprend une première partie et une seconde partie qui diffèrent l'une de l'autre au niveau de la matière et/ou de la structure.

8. Utilisation selon la revendication 7, dans laquelle les première et seconde parties diffèrent au niveau de la taille des pores et/ou de la taille du grain.

9. Utilisation selon l'une des revendications précédentes, dans laquelle l'élément à filtre acoustique (24) a une forme analogue à un tube.

10. Utilisation selon la revendication 9, dans laquelle l'élément à filtre acoustique (24) est pourvu d'au moins un canal (36) s'étendant de l'extrémité extérieure à l'extrémité intérieure de l'élément à filtre acoustique.

11. Utilisation selon l'une des revendications précédentes, dans laquelle la matière poreuse (28) a une taille de pores allant de 1 à 500 µm.

12. Utilisation selon l'une des revendications précédentes, dans laquelle la matière poreuse (28) a une porosité de 0,05 à 0,90.

13. Utilisation selon l'une des revendications précédentes, dans laquelle la matière poreuse (28) à un indice de structure de 1 à 12.

14. Utilisation selon l'une des revendications précédentes, dans laquelle la matière poreuse (28) a une résistance à l'écoulement par unité de longueur de 0,1 à 1000 Pa.s/m².
